Europäisches Patentamt

(19) European Patent Office

Office européen des brevets

(11) Publication number: **0 308 238**
A1

# (12) EUROPEAN PATENT APPLICATION

(21) Application number: 88308573.0

(22) Date of filing: 16.09.88

(51) Int. Cl.⁴: **A 61 K 37/02**
A 61 K 47/00

(30) Priority: 18.09.87 US 98817

(43) Date of publication of application:
22.03.89 Bulletin 89/12

(84) Designated Contracting States:
AT BE CH DE ES FR GB IT LI LU NL SE

(71) Applicant: ETHICON INC.
U.S. Route 22
Somerville New Jersey 08876 (US)

(72) Inventor: Finkenaur, Amy L.
10 Stevens Avenue
Somerville New Jersey 08876 (US)

Cohen, Jonathan M.
16 Pheasant Drive
Marlboro New Jersey 07746 (US)

(74) Representative: Jones, Alan John et al
CARPMAELS & RANSFORD 43 Bloomsbury Square
London, WC1A 2RA (GB)

(54) Stable lyophilized formulations containing growth factors.

(57) Stable lyophilized compositions comprising polypeptide growth factors and kits containing the compositions are provided. The compositions may contain extenders for lyophilization and may also contain water soluble polymers for imparting viscosity to a reconstituted solution.

EP 0 308 238 A1

## Description

## STABLE LYOPHILIZED FORMULATIONS CONTAINING GROWTH FACTORS

Background of the Invention

Throughout this disclosure, various publications, patents and patent applications are referenced. The disclosures of these publications, patents and applications in their entireties are hereby incorporated by reference into this disclosure in order to more fully describe the state of the art as known to those skilled therein as of the date of the invention described and claimed herein.

The present invention relates to stable lyophilized formulations containing polypeptide growth factors having human mitogenic activity, in particular, epidermal growth factor.

The human polypeptide growth factors are molecules that regulate the growth of normal human cells. Many human polypeptide growth factors have been identified and their structures determined. Those falling within this group are: epidermal growth factor (EGF), acidic and basic fibroblast growth factor (FGF), platelet-derived growth factor (PDGF), transforming growth factor-α (TGF-α), transforming growth factor-β (TGF-β), insulin-like growth factors (IFG-I and IGF-II), and nerve growth factor (NGF). Because of their ability to stimulate cell growth, the human polypeptide growth factors have been described as being useful in stimulating cell growth, the human polypeptide growth factors have been described as being useful in stimulating the wound healing process.

One of the better characterized growth factors is epidermal growth factor. Human EGF is a 53 amino acid polypeptide growth factor that has mitogenic activity for a number of kinds of cells, including epithelial and mesenchymal cells. Variants of the naturally occurring EGF molecule have been reported, such as the 52 amino acid gamma-urogastrone. EGF has also been reported to have angiogenic activity. Epidermal growth factor exhibits epidermal growth promoting activity and gastric acid secretion inhibiting activity, and is therefore useful as a medicament. It has been found that epidermal growth factor loses biological activity in the presence of moisture. This is disadvantageous because such loss of activity makes it impractical to store aqueous preparations of epidermal growth factor for extended periods of time. This invention provides a means for reducing or preventing the loss of activity of a polypeptide growth factor formulation, such as EGF, by providing stable lyophilized formulations containing these growth factors.

Many biological materials, which will rapidly deteriorate even in frozen solutions, can be kept in a viable state for long periods of time by lyophilization of the material. Lyophilization (also known as freeze drying) is a process of drying a composition in which water is sublimed from the composition after it is frozen. The particular advantages of this process are that biologicals and pharmaceuticals which are relatively unstable in an aqueous solution can be processed and filled into dosage containers in the liquid state, taking advantage of the relative ease of processing of a liquid; dried without elevated temperatures, thereby eliminating adverse thermal effects; and then stored in the dry state in which there are relatively few stability problems. Thus, the product can be stabilized against loss of biological activity to provide a long shelf life.

Lyophilization essentially consists of the following steps. First, an aqueous solution of the product is frozen at a temperature below its eutectic temperature in an evacuation chamber. The chamber is then evacuated, usually below 0.1 Torr. Ice is sublimed on a cold condensing surface at a temperature below that of the product, the condensing surface being within the chamber or in a connecting chamber. Then, heat is introduced to the product under controlled conditions, thereby providing energy for sublimation at a rate designed to keep the product below its eutectic temperature.

It is often impractical to design formulations based merely on the lyophilization of the bulk drug. This is so because usually the amounts of the drug used in the formulation will be very small. This is a problem because during the lyophilization process the drug can be pulled from the lyophilization container by the vacuum employed in the process. Furthermore, many polypeptides are relatively unstable when lyophilized in small concentrations. They can absorb to product packaging and lose activity. Many lyophilized pharmaceutical compositions rely on the use of a diluent or extender to increase the amount of solid present during the lyophilization process and thereby eliminate the problems associated with lyophilization of small amounts of bulk drug.

A lyophilized EGF preparation has been described in U.S. Patent No. 3,917,824 (Camble et al.) in which a lyophilized preparation was made containing mouse EGF and dextrose in a sterile sealed vial. The dextrose was added so that reconstitution of the lyophilized preparation formed an isotonic injectable solution.

Summary of the invention

The present invention provides a stable lyophilized composition containing a polypeptide growth factor having human mitogenic or angiogenic activity and a water soluble extender for lyophilization. The ratio of the weight of growth factor to weight of extender may be within the range of about 0.00001 to about 0.5. The water or moisture content of the lyophilized composition is preferred to be less than 1% by weight, although it may be as high as 5%. It is preferred that the growth factor is a human polypeptide growth factor and it is preferred that the human polypeptide growth factor is epidermal growth factor.

In one embodiment, the present invention provides a stable lyophilized composition containing a human polypeptide growth factor and a viscosity increasing, water soluble or water swellable, pharmaceutically acceptable polymer which is capable of imparting viscosity or increasing the viscosity of a reconstituted solution of the lyophilized composition. By varying the concentration of the gel forming polymer in a lyophilized cake, the viscosity of a reconstituted solution can be varied to any desired level. If the polymer is in the lyophilized cake, it may range in concentration (after reconstitution) from 0.1% to 10% by weight. The growth factor concentration in the reconstituted solution may be in the range 0.01-1000 micrograms/ml. The lyophilized solid may be a mixture of a solid diluent and a polymer, in which case the ratio of diluent to polymer may be about 3:1 (Diluent: polymer) by weight. The viscosity enhancing polymers are included in the lyophilized composition when a gel preparation or stabilized aqueous solution is desired after reconstitution. As used herein, reconstitution refers to restoring a dry lyophilized composition to its former aqueous form by the addition of an aqueous diluent.

Heretofore, human polypeptide growth factors have not been lyophilized with the appropriate components to prepare a formulated product suitable for use in human wound healing applications. The present invention provides lyophilized formulations and kits containing the compositions for use in human wound healing as well as stable lyophilized compositions that have a long shelf life. Lyophilized formulations provide the advantages of standardized physical form, standardized physico-chemical properties, chemical and biochemical integrity, increased biological viability and the capacity for rapid and total rehydration to an aqueous solution at the time of use.

## Detailed description of the Invention

It is important that the lyophilized compositions of the present invention have a very low water content, preferably less than 1% water by weight. The presence of water in the lyophilized compositions, in particular that of EGF, tends to decrease the biological activity of the growth factor. Biological activity may be measured in recognized bioassays, such as the EGF receptor binding assay described in EP-A-0 267 015 Additionally, stability of the growth factor may be measured by any suitable HPLC method. At one extreme, the composition would be a bone dry composition having 0% water. In practice, however, lyophilization of polypeptide solutions can result in lyophilized cakes having moisture contents that are as high as 10%.

The ratio of the weight of growth factor to the weight of extender used in the compositions of the present invention should be within the range of about 0.00001 to about 0.5. As used herein, the word extender means a water soluble, solid particulate diluent used for facilitating lyophilization by increasing the solid material present in the final lyophilized composition. The extender provides mechanical integrity to the lyophilized cake. The growth factor/extender ratio may vary depending on the amount of growth factor that is desired to be used in the final preparation. In those situations where a dilute aqueous solution of growth factor is ultimately desired, the growth factor/extender ratio in the lyophilized composition will be low. In those situations where the amount of growth factor is desired to be high, such as when the lyophilized preparation will be placed directly on a wound such as a burn, the growth factor/extender ratio in the lyophilized composition will be high. In a preferred embodiment, the growth factor/extender ratio in the lyophilized composition is from about 0.0001 to about 0.1.

The water soluble extenders suitable for use in the present invention can be any of the pharmaceutically acceptable essentially inert solid materials typically used for lyophilization in the formulation art. The extender should be non-toxic, it should not decrease the bioactivity of the growth factor and it should have no specific pharmacological action in the amount used. Such extenders include simple sugars, such as monosaccharides, including glucose, dextrose and the sugar alcohol mannitol; disaccharides, such as sucrose and lactose; polysaccharides such as dextran; amino acids such as glycine and alanine; short chain ($C_3$-$C_6$) tricarboxylic and dicarboxylic acids, such as citric acid and tartaric acid, respectively; and certain inorganic salts, such as the sodium or potassium phosphates. In a preferred embodiment, the extender is mannitol. In any final reconstituted solution of the present invention, the concentration of extender should be between about 0.1 and about 6.0% (w/v). Since growth factors such as EGF do not crystallize well, or not at all, and do not pack well by themselves in lyophilized form, the extenders enhance the packing of the lyophilized growth factor into a solid "cake".

The polypeptide growth factors referred to herein are those having human mitogenic or angiogenic activity selected from the group consisting of EGF, acidic-FGF, basic-FGF, PDGF, TGF-$\alpha$, TGF-$\beta$, angiogenin, NGF, IGF-I, IGF-II, or mixtures thereof. Any biologically active fragments or chemically synthesized derivatives of these growth factors are within the scope of the present invention. In addition to mitogenic activity, EGF, the FGF'S, the TGF's and angiogenin are reported to have angiogenic activity. It is preferred that the growth factor be prepared by recombinant DNA techniques. As used herein, the phrase "growth factor" does not include the so called hematopoietic growth factors such as erythropoietin and the colony stimulating factors.

As used in this application, EGF is intended to include the class of polypeptides that have biological activity similar to that exhibited by the natural human EGF polypeptide, as measured in recognized bioassays, such as the EGF receptor binding assay, and which have in common certain conserved amino acid residues and positioning of disulfide bonds, as discussed by Carpenter et al. in "Epidermal Growth Factor, its Receptor and Related Proteins", Experimental Cell Research, 164:1-10 (1986). Thus, EGF includes the human EGF produced by recombinant DNA techniques, mouse EGF isolated from the submaxillary glands of mice, rat EGF, and natural human EGF which may be isolated from human urine, and bioactive derivatives and related peptides of any of the foregoing, including precursors that are transformed into active EGF in situ by proteolytic processing. Human epidermal growth factor, including human EGF produced by recombinant DNA techniques, is preferred for use in the present invention. The amino acid sequence of human EGF is set forth in Urdea, M.S. et al., Proc. Natl. Acad. Sci. (USA), 80:7461-7465 (1983). Human EGF also refers to any naturally occurring human EGF variant, such as gamma-urogastrone.

In a further embodiment, the present invention provides a stable lyophilized composition which comprises a human polypeptide growth factor and a water soluble, pharmaceutically acceptable polymer capable of imparting viscosity or enhancing the viscosity of a reconstituted solution of the lyophilized composition. The polymer has the characteristics of being a hydrophilic gel having a high water content wherein the gel matrix permits the gel to serve as a growth factor carrier. Polymer containing lyophilized compositions may be prepared when the final reconstituted solution is desired to be a gel, or a more viscous aqueous solution or a stabilized aqueous solution. The desired viscosity ranges for reconstituted solutions of the various polymers will vary for each intended application on a patient. For ophthalmic applications, the viscosity range should be 1-5000 cps (centipoise). For use in the anterior chamber of the eye, the viscosity range should be 10,000-100,000 cps. For cutaneous or incisional wound healing applications, the viscosity range is preferred to be 1,000-12,000,00 cps. Viscosity values referred to herein are determined by the Brookfield method at room temperature, e.g. 22-26° C.

The viscosity enhancing polymer may be selected from the group consisting of vinyl polymers, polyoxyethylene-polyoxypropylene polymers or co-polymers (Pluronics®), polysaccharides, proteins, poly(ethyleneoxide), acrylamide polymers and derivatives or salts thereof. It is understood that poly(ethyleneoxide) includes polyethylene glycol. The vinyl polymers useful in the present invention may be selected from the group consisting of polyacrylic acid, polymethacrylic acid, polyvinyl pyrrolidone and polyvinyl alcohol. The polysaccharides useful in the present invention may be selected from the group consisting of cellulose or cellulose derivatives, glycosaminoglycans, agar, pectin, alginic acid, dextran, starch and chitosan. The glycosaminoglycans may be selected from the group consisting of hyaluronic acid, chondroitin, and related molecules. The proteins useful in the present invention may be selected from the group consisting of collagen, gelatin and fibronectin. The Pluronics® and polyethylene glycol cannot be lyophilized and therefore cannot be in the lyophilized cake. However, they can be in the solution used to reconstitute the cake.

The cellulose derivatives may include alkyl celluloses and hydroxyalkyl celluloses, for example, methyl cellulose, hydroxyethyl cellulose, carboxymethyl cellulose, hydroxypropylmethyl cellulose and hydroxypropyl cellulose.

The molecular weights of the polymers will depend on which application the polymer will be used for treating the patient. For example, for use in the anterior chamber of the eye, a moderately viscous gel is required and the molecular weight is selected to provide such a viscosity. Hyaluronic acid may have a molecular weight of about one million in a 1% solution for this application. Hydroxypropyl methyl cellulose may have a MW of 86,000 in a 2% solution for use in the anterior chamber. Polyacrylamide may have a molecular weight of three million in a 4.5% solution for anterior chamber use.

The gel forming materials used in the present invention may be water soluble polymers capable of forming a viscous aqueous solution or non-water soluble, but water swellable polymers (e.g. collagen), which can also form a viscous solution. Swellable polymers are those which absorb water rather than dissolve in water.

The cellulose derivatives are capable of stabilizing the polypeptide growth factors, in particular EGF, against loss of biological activity in an aqueous solution. Use of cellulose derivatives to stabilize growth factors against loss of biological activity is described in EP-A-0 267 015.

Gel formulations containing growth factors are described in co-pending European application No (ETH-714), entitled "Gel Formulations Containing Growth Factors", filed concurrently herewith.

Aqueous gel formulations are useful in ophthalmic and cutaneous wound healing. Gels have the advantage of adhering to a wound and conforming to irregular body or wound contours. Reconstituted gels may be applied directly to a wound site or as a coating on a compliant porous or microporous substrate to be applied to the wound site. Gels have the further advantages of having a high water content (which would keep the wound moist), an ability to absorb wound exudate, easy application to a wound and easy removal by washing. Gels have a cool feeling when applied to a wound and thus may increase patient comfort and acceptance.

The reconstituted gel formulations of the present invention provide a controlled delivery system for growth factors on a wound site. Controlled release refers to drug release sufficient to maintain a therapeutic level over an extended period of time, such as up to 24 hours or more, preferably in the range of 1-12 hours and more preferably 1-8 hours. Increased contact time of growth factors, in particular EGF, at the wound site has been reported to be necessary to achieve a significant increase in the rate of wound healing. The present gel formulations increase the contact time of the growth factor at the wound site and provide a sustained release dosage form. This is an important advantage because it permits less frequent application of the formulation to the wound and thereby permits less disturbance of the wound.

In another embodiment, the present invention provides a kit for preparing a growth factor solution. The kit comprises a first vial containing a lyophilized human polypeptide growth factor having mitogenic activity. Additionally, the kit comprises a second vial containing a buffered liquid diluent. The kit has the advantage of providing a stable formulation of the growth factor which has a long shelf life. A fresh stable solution of the growth factor can be prepared at any desired time by mixing the contents of the second vial with that of the first vial. The resulting solution may then be used immediately or it may be used over a several day period, i.e. when a multi-use container is prepared. If the solution is not used immediately, it is desirable to have one of the cellulose derivative stabilizers present in the liquid diluent so that the reconstituted aqueous solution will be stabilized against loss of biological activity. Additionally, a preservative to prevent microbial growth should be present in any multi-use container. In a preferred embodiment, the growth factor in the first vial is epidermal growth factor.

The lyophilized growth factor in the first vial may additionally contain a water soluble extender or a water soluble, viscosity enhancing polymer as described herein. The liquid diluent in the second vial may be pure water or may additionally contain a buffer for maintaining the pH of the solution between about 5.0 and about 8.0, preferably 7.0-8.0. The pH may depend on the particular preservative being used, if any. Suitable buffers are those selected from the group consisting of phosphate buffers, citrate buffers, borate buffers and acetate buffers.

An optional ingredient that may be included in the liquid diluent is a tonicity agent for making the aqueous solution isotonic and isosmotic. The tonicity agent may be present in a concentration of up to about 1.8% (w/v). Suitable tonicity agents are chloride salts, such as sodium chloride and potassium chloride. The extenders mentioned above such as mannitol, can also act as a tonicity agent as well as an extender and therefore the tonicity agent may be omitted from the formulation and more of the extender added to achieve a desired tonicity affect.

The liquid diluent may also contain a preservative, at a concentration of about 0.0002% to about 2.5% (w/v), the concentration depending on the particular preservative used. Suitable preservatives are thimerosal (0.002-0.1%), sorbic acid (0.05-2.5%), chlorobutanol (0.05-0.6%), and imidazolidinyl urea (0.01-0.1%). Preservatives of the paraben class, such as methyl (0.15-0.20%) and propyl (0.01-0.03%) parabens, are also suitable.

The liquid diluent in the kit of the present invention may contain one or more water soluble, viscosity increasing polymers as described herein. Polymers which are waxy, such as polyethylene glycol and the Pluronics®, are not suitable for being placed directly into the lyophilized cake because they have glass transition temperatures which are near or below the temperature used for lyophilization (35°C). Polymers near or below their glass transition temperature melt and flow during the lyophilization process, causing the cake structure to collapse with resultant cake being difficult to redissolve upon reconstitution. Thus, it is preferred that the polymer have a glass transition temperature which is substantially higher than 37°C if the polymer is desired to be placed in the cake. Otherwise, any of the polymers described herein may be used in the liquid diluent. In such a case, the lyophilized composition in the first vial would contain only growth factor and extender. Then, the polymer containing diluent is added to the vial containing the lyophilized cake to form a gel solution.

Any or all of the ingredients described as being in the liquid diluent of the second vial may alternatively be present in the lyophilized cake of the first vial, with the exclusion of those ingredients which are the volatile or noncomptabile polymers previously mentioned. Volatile ingredients, such as certain preservatives, are lost during lyophilization and therefore would have to be in the liquid diluent if desired to be used in a final reconstituted aqueous formulation. Thus, the lyophilized cake may contain any combination of growth factor, extender, polymer, buffer, tonicity agent or non-volatile preservative. In the case where all the additional ingredients are in the lyophilized cake, the liquid diluent may simply be water. In the case where the tonicity agent is not in the cake, the diluent may be saline.

A method for preparing an aqueous growth factor solution, comprises first preparing any of the lyophilized compositions of the present invention and then mixing the lyophilized composition with an aqueous diluent as described herein. The advantage of such a method is that it permits the growth factor to be stored in a stable form over an extended period of time and then freshly prepared in aqueous form (liquid or gel) when it is desired to be used. The growth factor concentration in the solution may be in the range of about 0.01 to about 1000 micrograms/ml. It is preferred that the growth factor concentration be about 1-500 micrograms/ml and more preferably 1-100 micrograms/ml.

A method for increasing the rate of healing a wound comprises contacting the wound directly with a wound healing effective amount of a lyophilized composition comprising a human polypeptide growth factor having mitogenic activity. The lyophilized powder containing the growth factor may be sprinkled directly on the wound in any manner, such as from a standard powder dispenser or from an aerosol spray powder dispenser. After the lyophilized powder is on the wound it may then be covered with a protective cream, gel or bandage in order to maintain the powder on the wound.

The lyophilized particulate delivery system of the present invention has the advantage of long term stability of the growth factor and ease of application. The lyophilized growth factor-extender composition may be ground into a very fine powder before applying to a wound. For those wounds which are "dry" type wounds, e.g. burns or donor sites, the powder should not dehydrate the wound. In a "wet" type wound, e.g. certain ulcers, dehydration by the powder is not an important concern.

Gelled systems containing growth factors may be lyophilized forming a foam sheet or pad. The dry foam may be added directly to the wound and covered with an appropriate dressing. Liquid exudate from the wound causes it to hydrate the foam thereby restoring the foam to the gel state thus permitting release of the growth factor to the target tissue. Such a system offes the sustained release benefits of a gel and the stability of a dry lyophilized state.

The lyophilized compositions of the present invention may be used to coat fibers of an absorbent gauze dressing to form a wound healing bandage which may then be placed on a wound. The wound healing bandage may be prepared by soaking an absorbent gauze dressing with a solution containing a human polypeptide growth factor having mitogenic activity and then lyophilizing the dressing to produce a bandage wherein the bandage fibers are coated with the growth factor in lyophilized powder form. The bandage can then be applied to the wound so that the growth factor in and on the fibers contacts the wound and stimulates cell growth to increase the rate of wound healing. After the bandage has been placed on the wound it may be soaked with water or any suitable aqueous solution to enhance the release of the lyophilized growth factor from the gauze onto the wound.

Reconstituted compositions of the present invention are useful in eyedrop formulations, salves for wound healing, gel formulations, foams and the like. Products which may include the lyophilized powders and reconstituted solutions of the present invention are liposome formulations, artificial skin, suture coatings, surgical staples and products for gastrointestinal indications, such as for use in inhibiting gastric acid secretion.

Wounds that may be healed using the compositions of the present invention are those which result from any accidental or medical injury which causes epithelial damage such as ophthalmic wounds, which result from corneal ulcers, radiokeratotomy, corneal transplants, epikeratophakia and other surgically induced wounds in the eye; and cutaneous wounds such as burn wounds, donor site wounds from skin transplants, and ulcers (cutaneous, decubitis, venous stasis and diabetic). Internal wounds may also be treated by the compositions of the present invention.

Methods of freeze drying are well known in the art and are set forth, for example, in Methods in Enzymology, Vol. 22, Pages 33-39, Academic Press, New York (1971); and in Freeze-Drying, E.W. Flosdorf, Rheinhold, New York (1949). In preparing the kits of the present invention, it is desirable to lyophilize the growth factor in the same vial in which it will be sold. An aqueous solution of the growth factor is added to the vial after filtering through a sterilizing filtration system, such as a 0.22 micron filter standardly used in sterilizing polypeptides. The contents of each vial may then by lyophilized and afterwards the vials capped and sealed under sterile conditions. A sterile final product is desirable when the product is going to be used for wound healing. In general the most useful container for a vial are the glass bottles standardly used for lyophilizing biological materials. Another suitable container is a two-compartment syringe wherein one compartment contains the lyophilized cake and the other compartment contains the aqueous diluent. After lyophilization is complete, the vacuum within the vials or ampules may be released by filling the system with an inert gas, stoppered in place using standard equipment and then crimp sealed. Such a method will ensure a sterile final product.

A variety of containers are suitable for lyophilization. A proper container should be able withstand the outside pressure when the container is sealed and stored under partial vacuum. The container should be made of a material that allows a reasonable transfer of heat from outside to inside. The size of the container should be such that the solution to be lyophilized occupies not more than 20% of the useful volume. For example, a 1 ml solution may be filled in a 5 ml vial or a 24 ml solution may be filled in a 120 ml vial. The vials may be for single use or multi-use application. The vials may be made of glass or plastic, e.g. polypropylene.

In preparing the kit of the present invention, the ultimate delivery system prepared from the kit must be sterile, non-antigenic and free of infectious agents, such as bacteria. Therefore, the kit must be prepared under aseptic conditions or the kit and ingredients must be sterilizable. Sterile filtering and autoclaving are suitable methods for this purpose. All packaging materials, such as glass and rubber may be sterilized by steam. The gels (before the growth factor is added) may also be sterilized by steam. The liquid and growth factors may be sterile filtered. Furthermore, for any container or vial which is to be used as a multi-use container, it is desirable to include a preservative in the container.

The following examples are presented to illustrate the subject invention. The invention is not to be considered limited by these examples, but only by the appended claims.

<u>EXAMPLE I</u>

A formulation for an eye drop was designed. The formulation was divided into a portion to be lyophilized and a liquid diluent. The formulation was divided as follows:

| Lyophilized Cake | Liquid Diluent |
|---|---|
| EGF | NaCl/tonicity agents |
| Mannitol/Extenders | Preservatives |
| Buffers (optional) | Buffers |
| | Polymers to increase viscosity |

The components of the lyophilized cake were dissolved in 2 ml of sterile water. The human EGF utilized was produced by recombinant techniques (Chiron Corp; Emeryville, Ca). Each 2 ml portion of this solution was placed in a 5 ml serum vial. The partially filled vials were placed on a tray in a lyophilization chamber and were frozen. The following lyophilization cycle was used to process the samples cited in this example (temperatures are the shelf temperatures, not the product temperatures);

1. -55°C for four hours at one atmosphere pressure.

2. -25°C for 14 hours at one atmosphere.

3. -55°C for 0.5 hours at full vacuum. The gauge monitoring the chamber pressure read 20 micrometers Hg during this time period.

4. The shelf temperature was raised to 22°C and held for 65 hours at full vacuum. The product temperature rose to the shelf temperature over a 12-hour period. The gauge monitoring chamber pressure read 60 micrometers Hg initially, falling to 20 micrometers Hg as the ice sublimed completely. Samples 2065-29A, B, and C were removed at this time.

5. Examples 2065-29 BX were further processed at 37°C for four hours at 20 micrometers Hg. At the end of this step, the remaining samples were removed.

Examples of several cake materials were prepared and the stability thereof tested at 37°C. The data and compositions of these formulations is set forth in Table 1. The liquid phase of these products would contain the cellulose derivative stabilizers which increase the stability of the formulation after reconstitution. Stability data was based on a dilution of 10 micrograms/ml.

Table 1

COMPOSITION AND STABILITY OF LYOPHILIZED CAKES AT 37°C

| Formulation | Composition | Day Number | | | | |
|---|---|---|---|---|---|---|
| | | 0 | 30 | 70[1] | 100 | 209 |
| 2065-29A | EGF 50µg mannitol 50 mg | 9.4 | 8.9 | 5.4 | 11.9 | 10.6 |
| 2065-29B | EGF 50µg mannitol 50 mg Na citrate 8.1mg Citric acid 1.5mg | 10.3 | 6.8 | 4.2 | 9.5 | 6.5 |
| 2065-29BX | Same as 29B above; Longer drying cycle | 11.2 | 8.0 | 3.5 | 10.4 | 9.3 |
| 2065-29C | EGF 50µg mannitol 50mg Na citrate 16.3mg Citric acid 3.0mg | 7.7 | 9.2 | 4.6 | 11.2 | N.A. |

[1] Data obtained on day 70 is low due to poor quality cells used for the receptor binding assay.

EXAMPLE 2

Formulations for lyophilized cakes containing EGF/mannitol and phosphate buffers were designed. Listed below are examples containing varying amounts of phosphate buffer. The cakes derived from these formulations may be reconstituted with an appropriate aqueous diluent, which may contain a tonicity agent and/or a polymer to increase viscosity and a preservative. Such a dilution will yield an efficatious solution suitable for topical use. The cakes may be alternately diluted with solutions containing polymers to form a gel preparation, as in Examples 3 and 4.

Table 2

| Formulation | Composition | Amount |
|---|---|---|
| 2065-102 A1 | NaH$_2$PO$_4$*H$_2$O | 9.0 mg |
| | Na$_2$HPO$_4$*7H$_2$O | 27.4 mg |
| | Mannitol | 50.0 mg |
| | EGF | 50.0 μg |
| 2065-102 B1 | NaH$_2$PO$_4$*H$_2$O | 4.5 mg |
| | Na$_2$HPO$_4$*7H$_2$O | 13.7 mg |
| | Mannitol | 50.0 mg |
| | EGF | 50.0 μg |
| 2065-102 C1 | NaH$_2$PO$_4$*H$_2$O | 2.25 mg |
| | Na$_2$HPO$_4$*7H$_2$O | 6.85 mg |
| | Mannitol | 50.0 mg |
| | EGF | 50.0 μg |
| 2065-102 D1 | NaH$_2$PO$_4$*H$_2$O | 1.13 mg |
| | Na$_2$HPO$_4$*7H$_2$O | 6.43 mg |
| | Mannitol | 50.0 mg |
| | EGF | 50.0 μg |
| 2065-102 E1 | NaH$_2$PO$_4$*H$_2$O | 0.0 mg |
| | Na$_2$HPO$_4$*7H$_2$O | 0.0 mg |
| | Mannitol | 50.0 mg |
| | EGF | 50.0 μg |

The formulations listed in Table 2 were dissolved in 2 ml portions of sterile water and filled into 5 ml serum vials. The vials were arranged on a tray and placed into the lyophilization chamber. The following is the lyophilization cycle shelf temperatures used to process the samples cited in this Example:

1. -40°C for 3 hours at one atmosphere.

2. -25°C for 14 hours at one atmosphere.

3. -40°C for 0.5 hours at full vacuum. The chamber pressure gauge read 20 micrometers Hg.

4. Shelf temperature was raised gradually to 20°C and maintained for 64 hours at full vacuum. The gauge monitoring chamber pressure read 60 micrometers Hg, gradually falling to 20 micrometers Hg as the ice in the samples completely sublimed.

5. Shelf temperature was increased to 37°C at full vacuum. The chamber pressure was monitored at 20 micrometers Hg. Samples were drawn at 5, 8 and 24 hours beyond the 64 hours at 20°C to determine the impact of increased time at 37°C on the dryness of the product.

6. Each sample was analyzed for moisture content. The percent moisture values are listed in Table 3.

Table 3

Percent Moisture Values for Listed Formulations

| Formulations | Time at 37 Degrees: | | |
|---|---|---|---|
| | 5 hours | 8 hours | 24 hours |
| 2065-102 A1 | 1.6 | 1.5 | 1.1 |
| 2065-102 B1 | 1.5 | 1.4 | 1.2 |
| 2065-102 C1 | 1.6 | 1.6 | 1.3 |
| 2065-102 D1 | 1.6 | NA | NA |
| 2065-102 E1 | 0.5 | 0.4 | 0.5 |

8

EXAMPLE 3

A hyaluronic acid gel was used to reconstitute the products of Example 2 to form an EGF/hyaluronic acid delivery system. In this example, a vial of 2065-102 A1 was reconstituted with hyaluronic acid gel. The gel contained 1% hyaluronic acid ("Amvisc"®; Medchem; Woburn, MA) in 0.9% saline. The lyophilized cake began to dissolve on contact with the gel. Complete dissolution was accomplished by gently inverting the vial to mix the contents for about one minute. The result was 5 ml of a uniform gel containing 50 micrograms of EGF, 9.0 mg of $NaH_2PO_4.H_2O$, 27.4 mg of $Na_2HPO_4.7H_2O$, 45 mg of NaCl 50 mg mannitol and 4.9g sterile water.

EXAMPLE 4

A thermogelling system was prepared using the products of Example 2 and reconstituting with an aqueous Pluronic® solution. In this Example, a vial of 2065-102 A1 was reconstituted with an 20% aqueous Pluronic® F127 solution. This solution has a low viscosity (i.e. is a liquid) when cool but gels between 20-30°C. Such a formulation can be made as a liquid which will gel when it contact a wound surface. To combine the components, 5 ml of Pluronic® solution was liquified by storing it in a 4°C refrigerator. The liquified portion was added to the vial using a syringe. The cake dissolved rapidly and with a few gentle inversions was completely dissolved within one minute. The resulting 5 ml of solution contained 50 micrograms EGF, 9.0 mg of $NaH_2PO4.H_2O$, 27.4 mg of $Na_2HPO_4.7H_2O$, 50 mg of mannitol, 900 mg of Pluronic F127 and 4.1 g of sterile water.

Example 5

In a preferred method, lyophilized EGF/mannitol was prepared in a 30 ml serum vial. A solution of mannitol (USP) in sterile water for irrigation (50 mg/ml) was mixed with 24 mg EGF. The sample formula was: 5g mannitol, 24 mg EGF and 100 ml water. 5 ml portions of this formula were aliquoted into 30 ml serum vials. Lyophilization stoppers were inserted half way into the vial necks. The vials were frozen to -40°C or lower. Full vacuum was applied with no heating until the chamber pressure fell below 100 micrometers Hg. The temperature of the heating shelf was then set to 37°C. Full vacuum was maintained and the vials were heated until the product temperature stabilized at final temperature for at least four hours. This final temperature was 32°C. It is preferred to be 30°C or higher. The time of the cycle was approximately 24 hours. The vials were stoppered under a nitrogen gas atmosphere.

EXAMPLE 6

Gelled systems containing EGF may be lyophilized and reconstituted with an aqueous diluent. A gel composed of 0.5% polyacrylic acid (Carbopol® 940, B.F. Goodrich) in water containing 50 micrograms EGF, 5% mannitol and pH adjusted to 7.0 with NaOH was prepared and lyophilized using the cycle in Example 1. Serum vials were filed with 2 ml portions of this gel prior to processing. When the resulting dry porous cake was recombined with 2 ml of water, a clear homogenous gel formed within 10 minutes. This gel was visually indistinguishable from gels that had not been lyophilized.

Example 7

In this example, a 2% solution of hydroxypropylmethyl cellulose (Methocel® E4M premium, Dow Chemical) was prepared in water. A 20 ml aliquot was removed and 200 micrograms EGF (in water 1 mg/ml) were added to make a final concentration of 10 micrograms/ml. A 10g portion of the gel mixture was placed in a flat bottomed glass dish (approximately 1 centimeter deep). The filled dish was frozen to -45°C and lyophilized to a maximum final temperature of 22°C. The result was a flexible spongy pad. The pad was found in kinetic studies to release 50% of its EGF in 3.3 hours.

The invention has been described herein with reference to certain preferred embodiments and examples. However, since obvious variations will appear to those skilled in the art, the invention is not to be considered limited thereto but only by the claims which follow.

**Claims**

1. A stable lyophilized composition comprising a polypeptide growth factor having human mitogenic or angiogenic activity and a water soluble or water swellable, pharmaceutically acceptable polymer capable of imparting viscosity to a reconstituted solution of the composition.

2. The composition of claim 1, wherein the polymer is a polysaccharide, vinyl polymer, protein, or acrylic polymer.

3. The composition of claim 2, wherein the vinyl polymer is polyvinyl alcohol or polyvinyl pyrrolidone.

4. The composition of claim 2, wherein the polysaccharide is a cellulose derivative selected from the group consisting of methyl cellulose, carboxymethyl cellulose or hydroxypropyl methylcellulose.

5. The composition of any one of claims 1 to 4, wherein the water content of the composition is less than about 5.0% by weight.

6. A kit, for preparing a growth factor solution, comprising:

a) a first vial containing a composition comprising a lyophilized polypeptide growth factor having human mitogenic or angiogenic activity; and

b) a second vial containing a buffered aqueous diluent.

7. The kit of claim 6, wherein the liquid diluent is buffered between pH 5.0 and pH 8.0.

8. The kit of claim 6 or claim 7, wherein the liquid diluent contains a water soluble, pharmaceutically acceptable viscosity enhancing polymer.

9. The kit of claim 8, wherein the polymer is a polysaccharide, a vinyl polymer, a protein, a Pluronic, a polyethylene glycol or an acrylic polymer.

10. The kit of any of claims 6 to 9, wherein the liquid diluent additionallly contains a preservative.

11. The kit of claim 10, wherein the preservative is thimerosal, sorbic acid, chlorobutanol, imidazolidinyl urea or a paraben.

12. The kit of any one of claims 6 to 11, wherein the diluent contains a phosphate buffer, a citrate buffer, a borate boffer or an acetate buffer.

13. The kit of any one of claims 6 to 12, wherein the vials are capable of withstanding lyophilization pressures and are capable of transferring heat from outside the vial to the inside of the vial.

14. The kit of any one of claims 6 to 13, wherein the vials are aseptically filled and sealed.

15. The kit of any one of claims 6 to 14, wherein said first vial contains a composition according to any one of claims 1 to 5.

16. The composition of any one of claims 1 to 5 or the kit of any one of claims 6 to 15, wherein the growth factor-containing composition additionally comprises a water soluble extender for lyophilisation.

17. The composition or kit of claim 16 wherein the ratio of growth factor to extender is from 0.00001 to 0.5 by weight.

18. The composition or kit of claim 16 or claim 17, wherein the extender is a monosaccharide, a disaccharide a polysaccharide, an amino acid, a short chain (C1 - C3) tricarboxylic acid or an inorganic salt.

19. The composition or kit of any one of claims 1 to 18, wherein the growth factor is epidermal growth factor, transforming grow factor-alpha, transforming growth factor-beta, acidic fibroblast growth factor, basic fibroblast growth factor, nerve growth factor, insulin-like growth factor, a biologically active fragment thereof, or a mixture thereof.

20. The composition or kit of claim 19, wherein the growth factor is epidermal growth factor or a biologically active fragment thereof.

21. A method for preparing an aqueous growth factor solution, comprising:

a) preparing the lyophilized composition of any one of claims 1 to 5 and 16 to 20; and

b) mixing the lyophilized composition with an aqueous diluent.

22. The composition or kit of any one of claims 1 to 20 for use in increasing the rate of healing a wound.

| | **DOCUMENTS CONSIDERED TO BE RELEVANT** | | EP 88308573.0 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X | US - A - 4 444 760 (K.A.THOMAS) . <br><br> * Column 3, lines 43-46, 55-56; column 4, lines 38-64; column 5, lines 42-52 * <br> -- | 1,2,4, 22 | A 61 K 37/02 <br><br> A 61 K 47/00 |
| X | EP - A2/A3 - 0 193 917 (AMERICAN CYANAMID CORP.) <br><br> * Claims 1,2,6 * <br> -- | 1,2,6 | |
| A | WO - A1 - 79/00 515 (THE TRUSTEES OF BOSTON UNIVERSITY) <br><br> * Claims 1,2,3,7-11 * <br> ---- | 1 | |

| TECHNICAL FIELDS SEARCHED (Int. Cl.4) |
|---|
| A 61 K 37/00 <br><br> A 61 K 31/00 <br><br> A 61 K 47/00 <br><br> A 61 K  9/00 |

The present search report has been drawn up for all claims

| Place of search | Date of completion of the search | Examiner |
|---|---|---|
| VIENNA | 07-12-1988 | DUNGLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82